Europäisches Patentamt
European Patent Office
Office européen des brevets

(19) (11) Veröffentlichungsnummer: **0 262 348 A2**

# EUROPÄISCHE PATENTANMELDUNG

(21) Anmeldenummer: 87111456.7

(22) Anmeldetag: 07.08.87

(51) Int Cl.4: C07D 295/02 , C07D 211/14 , C07D 265/30

(30) Priorität: 02.10.86 DE 3633520

(43) Veröffentlichungstag der Anmeldung: 06.04.88 Patentblatt 88/14

(84) Benannte Vertragsstaaten: BE CH DE FR GB IT LI NL

(71) Anmelder: HÜLS AKTIENGESELLSCHAFT
Patentabteilung / PB 15 - Postfach 13 20
D-4370 Marl 1(DE)

(72) Erfinder: Kaufhold, Manfred, Dr.
Jasminweg 20
D-4370 Marl(DE)

## (54) Verfahren zur Herstellung von N-substituierten Morpholin- und Piperidin-Derivaten.

(57) Zur Herstellung von N-substituierten Morpholin-und Piperidin-Derivaten der Formel (1) lagert man zunächst Bromwasserstoff peroxidkatalysiert an gegebenenfalls p-Alkyl-substituiertem Methallylbenzol (2) bzw. an einem Gemisch aus gegebenenfalls p-Alkyl-substituiertem Methallylbenzol (alpha-Olefin) (2) und dem entsprechenden beta-Isomeren, dem gegebenenfalls p-Alkyl-substituiertem Isobutenylbenzol (3) an und setzt die erhaltene Bromverbindung(en) (4 bzw. 5) anschließend mit Morpholin oder Piperidin bzw. deren Derivaten (6 und 7) um.

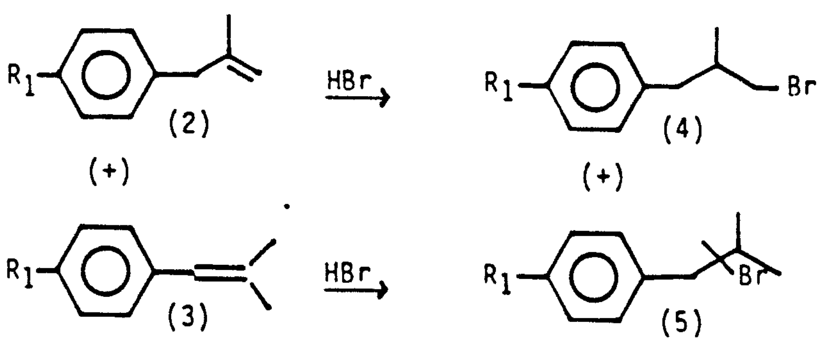

(2) (4)
(+) (+)
(3) (5)

$R_1$ = H, C1-C4-Alkyl wie Methyl-, Ethyl-, Isopropyl-, tert-Butyl-

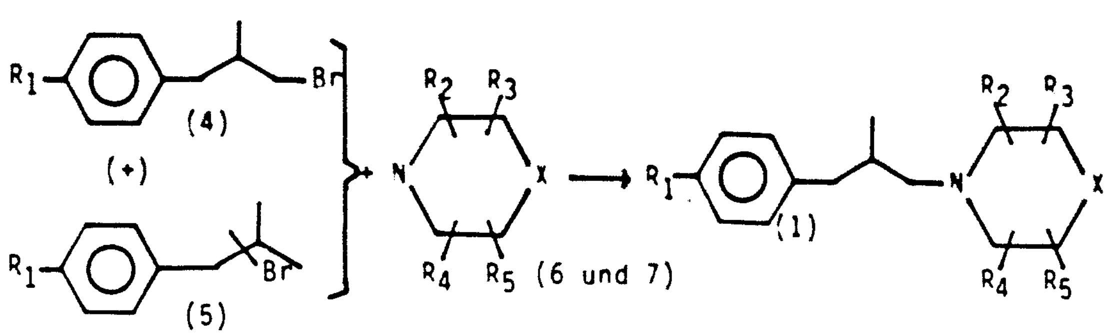
(4) (+) (5) (6 und 7) (1)

$R_2$, $R_3$, $R_4$, $R_5$ = Wasserstoff, Methyl oder Ethyl,
6 → X = O

EP 0 262 348 A2

BAD ORIGINAL

Xerox Copy Centre

7 → X = $CH_2$

# Verfahren zur Herstellung von N-substituierten Morpholin-und Piperidin-Derivaten

Die Erfindung betrifft ein Verfahren zur Herstellung von N-substituierten Morpholin-und Piperidin-Derivaten der Formel (1) durch peroxidkatalysierte Bromwasserstoffanlagerung an Methallylbenzol (2) bzw. ein p-alkylsubstituiertes Methallylbenzol (2) gegebenenfalls in Gegenwart des entsprechenden isomeren Isobutenylbenzols (3) bzw. p-alkylsubstituierten Isobutenylbenzols (3) und anschließende Umsetzung der Bromverbindung (4) bzw. der Verbindungen (4) und (5) mit Morpholin oder Piperidin, bzw. deren Derivaten (6 und 7)

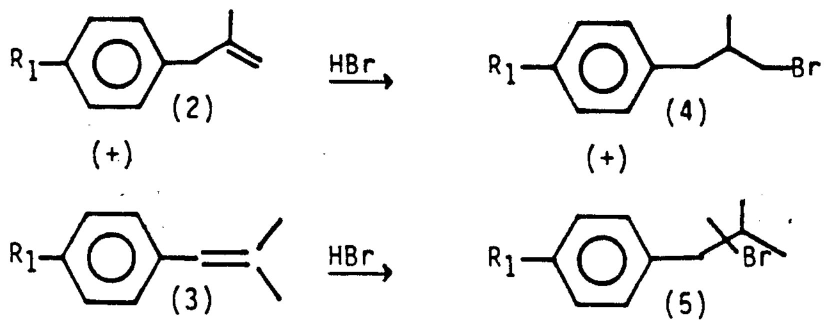


(2) (+) (3) (4) (+) (5)

$R_1$ = H, C1-C4-Alkyl wie: Methyl-, Ethyl-, Isopropyl-, tert-Butyl-

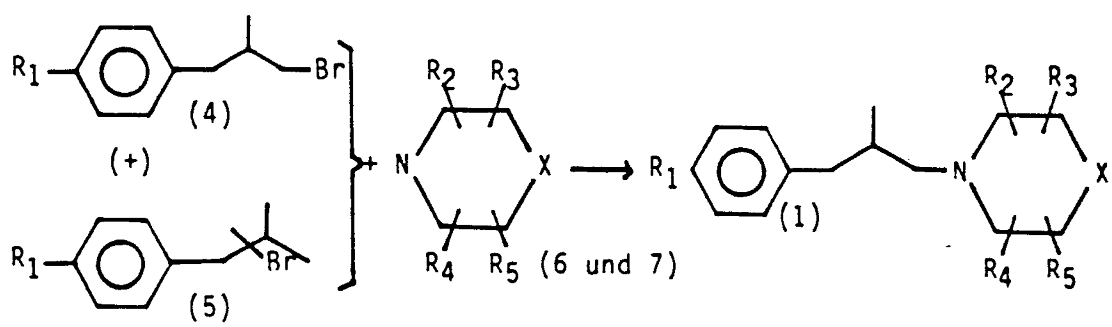

(4) (+) (5) (6 und 7) (1)

$R_2$, $R_3$, $R_4$, $R_5$ = Wasserstoff, Methyl oder Ethyl;
6 → X = 0
7 → X = $CH_2$

Synthesen von N-substituierten Morpholin-und Piperidin-Derivaten der Formel 1 sind bekannt. Die DE-OS 26 56 747 beschreibt ein Verfahren zur Herstellung von Morpholinderivaten, bei dem 3-p-tert.-Butylphenyl-2-methylpropanal mit 2,6-Dimethylmorpholin in Gegenwart von Ameisensäure umgesetzt wird. Nachteilig bei diesem und ähnlichen Verfahren ist der Einsatz des kostspieligen 3-p-tert.-Butyl-phenyl-2-methylpropanals das aus dem technisch schwer zugänglichen p-t-Butylbenzaldehyd durch Aldolisierung mit Propionaldehyd und anschließende selektive Hydrierung der Doppelbindung im Aldolisierungsprodukt hergestellt wird.

In der EP-PS 0 005 541 wird gezeigt, daß substituierte Morpholin-und Piperidin-Derivate mit Hilfe von Bromiden, die der Struktur (4) entsprechen, leicht zugänglich sind (siehe hier Beispiel 10). Die Bromide werden aber aus den entsprechenden Alkoholen mit Phosphortribromid synthetisiert, die ihrerseits wieder in einer vielstufigen Synthese ausgehend vom technisch schwer zugänglichen p-t-Buylbenzaldehyd hergestellt werden. Alle bekannten Verfahren zur Herstellung der in para-Stellung substituierten Morpholin-und Piperidin-Derivate (1) gehen also von den teuren und technisch schwer zugänglichen p-Alkylbenzaldehyden z. B. p-tert.-Butylbenzaldehyd aus. Wünschenswert wäre ein Verfahren, mit dem man die Bromverbindung (4) ausgehend von preisgünstigen Chemikalien in üblichen technischen Apparaturen herstellen könnte.

Es besteht ein großes Interesse an einem derartigen Verfahren, nach dem man bei geringem technischen Aufwand und ohne den Einsatz von teuren Reagenzien N-substituierte Morpholin-und Piperidin-Derivate herstellen kann, weil diese Produkte z. B. als Fungizide eine große wirtschaftliche Bedeutung haben.

Die sich hieraus ergebende Aufgabe wird erfindungsgemäß entsprechend den Angaben der Patentansprüche gelöst.

Überraschenderweise erhält man N-substituierte Morpholin-und Piperidin-Derivate der allgemeinen Formel 1 in guten Ausbeuten von ca. 80 bis 90 % und mit hoher Reinheit von 97 bis 98 % nach gaschromatographischer Analyse, indem man an Methallylbenzol bzw. an ein p-Alkyl-substituiertes Methallylbenzol (2) Bromwasserstoff in Gegenwart von Peroxiden addiert und die entstandene Bromverbindung (4) mit Morpholin oder Piperidin bzw. deren Derivaten umsetzt.

Dieser Effekt war nicht zu erwarten, da die Bromwasserstoffaddition eine Reihe von Nebenverbindungen liefert, die ebenfalls mit Morpholin oder Piperidin bzw. deren Derivaten weiter reagieren und zu Verunreinigungen im gewünschten Produkt führen sollten. An die Reinheit des eingesetzten Methallylbenzols (2) werden keine hohen Forderungen gestellt.

Ferner wurde überraschenderweise gefunden, daß der Gehalt an dem entsprechenden isomeren beta-Olefin, dem Isobutenylbenzol bzw. dem p-Alkyl-substituierten Isobutenylbenzol (3), im Methallylbenzol beliebig hoch sein kann, ohne daß dadurch die Reinheit im Endprodukt verringert wird d. h. man kann ein Olefingemisch von (2) und (3) einsetzen und erhält im Endeffekt nur das Umsetzungsprodukt von (2).

Es ist also ein großer Vorteil des erfindungsgemäßen Verfahrens, daß man technische Olefingemische einsetzen kann wie sie z. B. bei der thermischen Zersetzung von Neophylchlorid (= 2-Methyl-2-phenylpropylchlorid) bzw. p-Alkyl-substituierten Neophylchloriden anfallen (siehe US-PS 2 454 779). Bei dem Verfahren dieser Patentschrift erfolgt die Spaltung der Chlorverbindungen in Gegenwart von Alkalisalzen von Carbonsäuren und dadurch bedingt liegt das Isomerenverhältnis alpha : beta-Olefin bei 2 : 1 bis 1 : 1.

Als weiterer überraschender Vorteil des erfindungsgemäßen Verfahrens wurde gefunden, daß das aus dem Gemisch von tertiären und sekundären Bromverbindungen (5) bei der Umsetzung mit den Stickstoffverbindungen (6 und 7) entstehende Olefin nicht wie zu erwarten ein Isobutenylbenzol ist, sondern ein Gemisch an alpha-und beta-Olefin in ungefährem Verhältnis alpha : beta, wie 3 : 4. Dieses Gemisch kann wieder mit Bromwasserstoff umgesetzt und auf diese Weise direkt genutzt werden, was für die Wirtschaftlichkeit des Verfahrens von großem Nutzen ist.

Zur praktischen Durchführung ist folgendes wichtig:

Die Anlagerung von Bromwasserstoff an das Einsatzolefin erfolgt bei 0 bis 20 °C in Gegenwart von einem Peroxid z. B. Dibenzoylperoxid und einem unpolaren Lösemittel wie z. B. Hexan wie für andere Olefine in der Literatur beschrieben: "Methoden der Organischen Chemie", Houben-Weyl, Georg Thieme Verlag Stuttgart (1960) Band V/4 Seite 111. Nach dem Abdestillieren des Lösemittels wird das rohe Umsetzungsprodukt aufdestilliert oder direkt weiterverarbeitet. Dazu wird das Morpholin oder Piperidin bzw. deren Derivate vorgelegt, auf 50 bis 200 °C, vorzugsweise 100 °C bis zur Rückflußtemperatur der jeweiligen Stickstoffverbindung (6 und 7) erwärmt und die Bromverbindung (4) bzw. (4) und (5) zugegeben.

Als Morpholin-bzw. Piperidin-Derivate sindcis-2.6-Dimethyl-, -trans-2.6-Dimethyl-und cis-2.6-Diethyl-, -trans-2.6-Diethyl-morpholin usw. geeignet. Das Molverhältnis Stickstofffverbindung zur Bromverbindung beträgt 2 : 1 bis 20 : 1 vorzugsweise 2,1 : 1 bis 5 : 1.

Hierbei dient 1 Mol der Stickstoffverbindung zum Binden des gebildeten Bromwasserstoffs. Je nach Temperatur beträgt die Reaktionszeit einige Minuten bis zu 3 Stunden.

Nach der Umsetzung wird die überschüssige Stickstoffverbindung abdestilliert, Natronlauge zugegeben, um aus dem Hydrobromid weitere Stickstoffverbindung freizusetzen und diese ebenfalls abzudestillieren. Gegebenenfalls wird ein Lösemittel zugegeben wie z. B. Toluol, Xylol, Ethylbenzol, Diethylbenzol usw. und das gebildete Natriumbromid mit Wasser ausgewaschen. Die Ölphase wird destillativ aufgearbeitet.

Die folgenden Beispiele sollen das erfindungsgemäße Verfahren näher erläutern.

Beispiel 1 a

Man benutzt eine Glasapparatur, die aus einem Dreihalskolben mit Gaseinleitunsrohr, Rührer, Thermometer, Tropftrichter und Rückflußkühler besteht.

Man setzt ein
198 g (= 1,5 Mol) Methallylbenzol (98,5 prozentig)
666 g Hexan
10,8 g Dibenzoylperoxid (70 prozentig)

Die Lösung dieser Substanzen wird mit kaltem Wasserbad auf 10 °C gekühlt und bis zur Sättigung gasförmiger Bromwasserstoff eingeleitet.

Nach Abdestillieren des Lösemittels bei Normaldruck wird das Reaktionsprodukt bei 0,5 bis 0,8 mbar destilliert. Siedebereich 65 bis 72 °C; Destillatmenge 291 g. Gehalt nach GC-Analyse: 91,9 %. Ausbeute an 1-Phenyl-2-methyl-3-brom-propan 83,7 % d. Th., bezogen auf Einsatz.

Beispiel 1 b

Man benutzt eine Glasapparatur, die aus einem Dreihalskolben mit Rührer, Thermometer, Tropftrichter und aufgesetzter Destillationskolonne mit Destillationsvorrichtung besteht.

Man setzt ein
345,6 g (= 3 Mol) cis-2,6-Dimethylmorpholin (95,7 prozentig)
213,1 g (= 0,919 Mol) 1-Phenyl-2-methyl-3-brompropan (91,9 prozentig) aus Beispiel 1 a

Das 2,6-Dimethylmorpolin wird am Rückfluß zum Sieden erhitzt, wobei sich eine Temperatur von 142 °C einstellt. Innerhalb einer halben Stunde wird die Bromverbindung zugetropft und noch 2 Stunden am Rückfluß zum Sieden erwärmt. Hierbei steigt die Temperatur auf 158 °C an.

Dann wird auf 112 °C abgekühlt und 88 g (= 1,1 Mol) Natronlauge (50 %ig) zugegeben. Nun wird cis-2,6-Dimethylmorpholin abdestilliert und in dem Maße, wie Destillat anfällt, werden 300 g Diethylbenzol als Löse-bzw. Suspensionsmittel zugegeben und dieses andestilliert. Danach wird abgekühlt, das gebildete Natriumbromid mit Wasser ausgewaschen und die Ölphase destillativ aufgearbeitet.

Bei 1,3 mbar fallen in einem Siedebereich von 121 bis 123 °C 201 g Destillat mit einem Gehalt von 98,0 % an, das nach NMR-Spektrum das erwartete Morpholinderivat mit der Struktur (1) ist. Die Ausbeute beträgt 86,3 %, bezogen auf Einsatz.

Beispiel 2 a

Man benutzt die in Beispiel 1 a beschriebene Glasapparatur und setzt an Stelle von Methallylbenzol Isobutenylbenzol ein. Die Durchführung erfolgt so wie im Beispiel 1 beschrieben, nur daß wegen der geringen thermischen Stabilität auf eine Destillation der Bromverbindungen verzichtet wird. Nach Abdestillieren des Lösemittels verbleiben 294 g Rückstand. Nach NMR-Analyse ist das Hauptprodukt zu 80 bis 90 % 1-Phenyl-2-methyl-2-brompropan und die GC-Analyse zeigt einen Hauptpeak mit 87,5 %. Die Ausbeute an dieser tertiären Bromverbindung errechnet sich zu 80,5 % d. Th., bezogen auf Einsatz.

Beispiel 2 b

Man benutzt die in Beispiel 1 b beschriebene Glasapparatur und setzt an Stelle der hier aufgeführten primären Bromverbindung die tertiäre Bromverbindungen, die bei Beispiel 2 a erhalten wurde. Die Durchführung erfolgt wie im Beispiel 1 b beschrieben.

Bei der destillativen Aufarbeitung fällt im Gegensatz zum Ergebnis bei Beispiel 1 b im interessanten Siedebereich kein Produkt an, sondern nur Leichtsieder.

Beispiel 3 a

Man benutzt die in Beispiel 1 a beschriebene Glasapparatur und setzt an Stelle von Methallylbenzol p-tert-Butylmethallylbenzol mit einem Gehalt von 98,0 % und einem Gehalt an p-tert-Butylisobutenylbenzol von 0,4 % ein.

Die Durchführung erfolgt wie im Beispiel 2 beschrieben. Nach Abdestillieren des Lösemittels enthält der Destillationsrückstand nach GC-Analyse einen Hauptbestandteil zu 88,0 %, bei dem es sich nach NMR-Spektrum um 3-(p-tert-Butylphenyl)-2-methyl-1-brompropan handelt. Die Ausbeute an dieser Bromverbindung errechnet sich zu 90,5 %, bezogen auf Einsatz.

Beispiel 3 b

Man benutzt die in Beispiel 1 b beschriebene Glasapparatur und setzt an Stelle der hier aufgeführten Bromverbindung das Produkt ein, das bei Beispiel 3 a erhalten wurde. Die Durchführung erfolgt wie im Beispiel 1b beschrieben.
Die destillative Aufarbeitung liefert in einem Siedebereich von 149 bis 155 °C bei 0.8 mbar das gewünschte Morpholinderivat cis-4-[3-(p-tert.-Butylphenyl)-2-methylpropyl]-2.6-dimethylmorpholin, dessen Struktur (1) durch NMR-Spektrum bewiesen wird, in 98.2 %iger Reinheit. Die Ausbeute an (1) beträgt 89.4 % d. Th., bezogen auf Einsatz.

Beispiel 4 a

Man benutzt die in Beispiel 1 a beschriebene Apparatur und setzt an Stelle von Methallylbenzol ein Gemisch ein, das 97.6 %ig ist und zu 71.2 % aus p-tert.-Butylmethallylbenzol und zu 26.4 % aus p-tert.-Butylisobutenylbenzol besteht.
Die Durchführung erfolgt wie im Beispiel 2 a beschrieben. Der lösemittelfreie Destillationsrückstand enthält nach GC-Analse zwei Hauptbestandteile mit Gehalten von 31.6 und 57.7 %. Nach NMR-Spektrum ist der Hauptbestandteil 3-(p-tert.-Butylphenyl)-2-methyl-1-brompropan und der Nebenbestandteil die entsprechende tertiäre Bromverbindung.

Die Gesamtausbeute an Bromverbindungen ist 86.8 % d. Th., bezogen auf Einsatz und die Ausbeute an primärem Bromid ist 76.9 % d. Th., bezogen auf den Anteil an eingesetztem alpha-Olefin.

Beispiel 4 b

Man benutzt die in Beispiel 1 b beschriebene Glasapparatur und setzt an Stelle der hier aufgeführten Bromverbindung das Produktgemisch ein, das bei Beispiel 4 a erhalten wurde. Die Durchführung erfolgt wie im Beispiel 1 b beschrieben. Die Reinheit des destillierten Morpholinderivats liegt bei 98.3 %.
Die Ausbeute an cis-4-[3-(p-tert.-Butylphenyl)-2-methylpropyl]-2.6-dimethylmorpholin, (1) beträgt 75.8 % d. Th., bezogen auf eingesetztes p-tert.-Butylmethallylbenzol bei Beispiel 4 a, oder anders ausgedrückt: 1.068 Mol dieses alpha-Olefins ergeben 0.81 Mol Morpholinderivat. Daneben fallen 0.22 Mol alpha-Olefin und 0.29 Mol beta-Olefin an, während nur 0.40 Mol beta-Olefin bei Beispiel 4 a eingesetzt wurden. Ein geringer Teil der primären Bromverbindung ergibt also Olefine.

Beispiel 5

Man benutzt die in Beispiel 1 b beschriebene Glasapparatur und setzt an Stelle des hier aufgeführten cis-2.6-Dimethylmorpholins 3 Mol Piperidin ein. Die Durchführung erfolgt wie im Beispiel 1 b beschrieben. Es stellen sich beim Sieden am Rückfluß im Reaktor Temperaturen von 98 bis 104 °C ein, und während der Zugabe der Bromverbindung steigt die Temperatur langsam auf 113 °C.
Das gebildete Piperidinderivat mit der Struktur 1 siedet bei 128 bis 130 °C bei 2.7 mbar. Die Reinheit des Destillats liegt bei 98.5 % und die Ausbeute beträgt 79 %, bezogen auf Einsatz.

**Ansprüche**

1. Verfahren zur Herstellung von N-substituierten Morpholin-und Piperidin-Derivaten der Formel (1) dadurch gekennzeichnet,
daß man Bromwasserstoff peroxidkatalysiert an gegebenenfalls p-Alkyl-substituiertem Methallylbenzol (2) bzw. an einem Gemisch aus gegebenenfalls p-Alkyl-substituiertem Methallylbenzol (Alpha-Olefin) (2) und dem entsprechenden Beta-Isomeren dem gegebenenfalls p-Alkyl-substituiertem Isobutenylbenzol (3) anlagert und die erhaltene Bromverbindung(en) (4 bzw. 5) anschließend mit Morpholin oder Piperidin bzw. deren Derivaten (6 und 7) umsetzt.

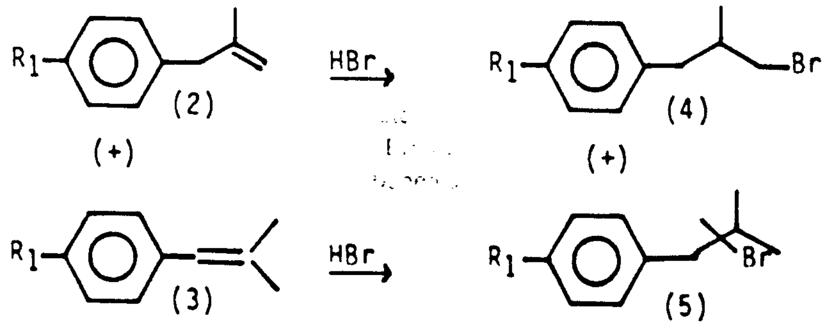


(2) (+) (3) (4) (+) (5)

C1-C4-Alkyl wie
$R_1$ = H, Methyl-, Ethyl-, Isopropyl, tert-Butyl

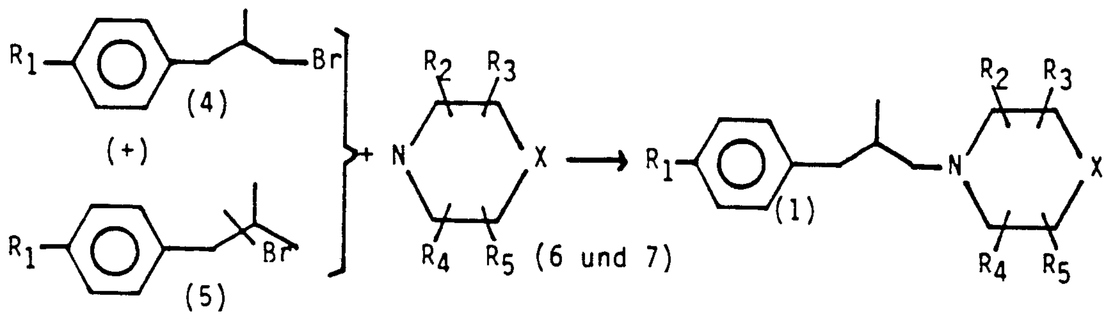

(4) (+) (5) (6 und 7) (1)

$R_2$, $R_3$, $R_4$, $R_5$ = Wasserstoff, Methyl oder Ethyl;
6 → X = O
7 → X = $CH_2$

2. Verfahren nach Anspruch 1,
dadurch gekennzeichnet,
daß man die Bromverbindung mit Morpholin oder Piperidin bzw. deren Derivaten bei Temperaturen von 50 bis 200 °C, vorzugsweise von 100 °C bis zur Rückflußtemperatur der eingesetzten Moprholin-oder Piperidin-Verbindung umsetzt.

3. Verfahren nach Anspruch 1 und 2,
dadurch gekennzeichnet,
daß man ein Molverhältnis Morpholin-oder Piperidin-Verbindung zu Bromverbindung 2 : 1 bis 20 : 1, vorzugsweise 2.1 : 1 bis 5 : 1, einsetzt.

4. Verfahren nach einem der Ansprüche 1 bis 3,
dadurch gekennzeichnet,
daß man als Morpholin-oder Piperidin-Verbindung Morpholin, 2,6-Dimethylmorpholin, -2,6-Diethylmorpholin, Piperidin, 2,6-Dimethylpiperidin, 2,6-Diethylpiperidin einsetzt.